# EUROPEAN PATENT SPECIFICATION

(11) **EP 0 407 818 B1**
(45) Date of publication and mention of the grant of the patent: **15.03.1995**
(21) Application number: 90112297.8
(22) Date of filing: 27.06.1990
(51) Int. Cl.: G01N 31/00, G01N 33/00

(54) **Hydrogen Gas Analyzer**
Wasserstoffprüfgerät
Appareil pour analyser le gaz hydrogène

(30) Priority: 12.07.1989 JP 81897/89 U
(43) Date of publication of application: 16.01.1991
(73) Proprietor: HORIBA, LTD., Minami-ku Kyoto (JP)
(72) Inventor: Koike, Hideki, Fusimi-ku, Kyoto (JP); Takeda, Kenji, Minami-ku, Kyoto (JP); Miyatake, Kimio, Kamikyo-ku, Kyoto (JP)
(74) Representative: TER MEER STEINMEISTER & PARTNER GbR

(56) References cited:
- EP-A- 0 107 341
- EP-A- 0 310 053
- PATENT ABSTRACT OF JAPAN,Vol.1,Nr.92,25.August 1977, pag.2415 E77;&JP - A 52 26896

## Description

The present invention relates to a hydrogen gas analyzer as claimed in the preamble of claim 1.

Such kind of hydrogen gas analyzer is already known in which a sample gas SG and an assistant fuel gas J is introduced into a catalytic tank, respectively, to burn said sample gas SG, and in which a moisture of the resulting gas is quantitatively determined to measure a concentration of a hydrogen gas contained in said sample gas SG.

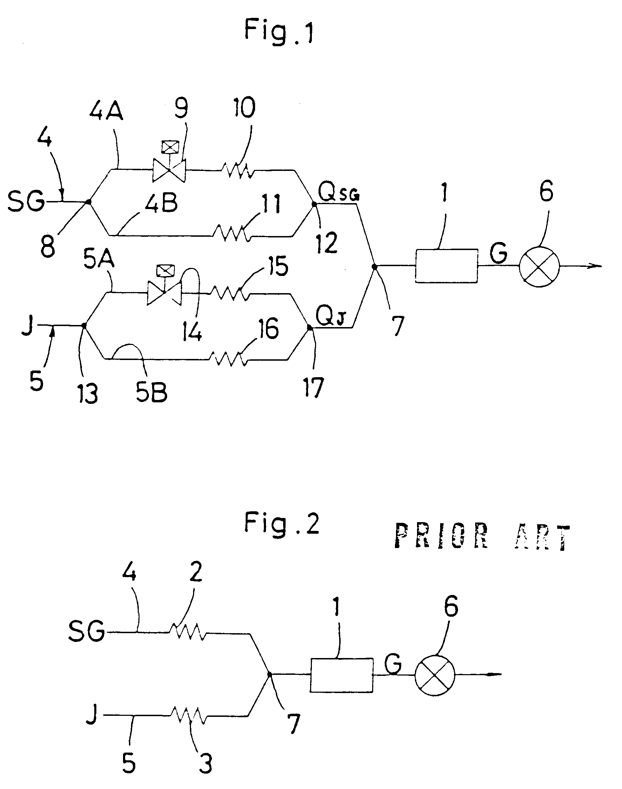

Said known gas analyzer is shown in Fig. 2 and is used as an apparatus for measuring the concentration of a hydrogen (H₂) gas contained in a combustion exhaust gas.

Referring to Fig. 2, reference numeral 1 designates a catalytic tank with an oxydation catalyst housed therein, said tank 1 is connected at the input side thereof with a sample gas supply passage 4 and an assistant fuel gas supply passage 5 provided with capillaries 2, 3 for regulating a flow rate, respectively. The passages 4, 5 are arranged in parallel to each other. A water meter 6, e.g. an infrared gas analyzer, is connected to the output side of the tank 1. Reference numeral 7 designates a confluence of said both supply passages 4, 5.

The combustion exhaust gas as the sample gas SG and air as the assistant fuel gas J are introduced into the catalytic tank 1 through the sample gas supply passage 4 and the assistant fuel gas supply passage 5, respectively, to burn the sample gas SG. Water (H₂O) in the resulting gas G is quantatively determined by means of the water meter 6 to measure the concentration of the hydrogen gas contained in said combustion exhaust gas.

However, if the combustibility of said fuel is deteriorated, H₂, CO (carbon monoxide) and HC (hydrocarbons) are contained in the combustion exhaust gas produced by the combustion of petroleum refinery fuels in an increased quantity. In such a case, if the concentration of H₂ in the combustion exhaust gas (sample gas SG) is measured by means of the hydrogen gas analyzer having the above described construction, H₂O is produced also by the combustion of HC coexisting in said sample gas SG, so that the result of the quantitative determination of H₂O by means of the water meter 6 includes not only H₂O resulting from the combustion of H₂ but also H₂O resulting from the combustion of HC, that is it is interferentially influenced by HC, so that the measurement can not be accurately conducted.

In order to avoid the above described interferential influence by HC it has already been suggested to maintain the catalytic tank 1 in Fig. 2 at a lower temperature, at which HC is difficult to burn.

In such a case, no buming of HC occurs due to the lower temperature of the catalytic tank] at the beginning. However, CO coexisting in the sample gas SG is burnt together with H₂. Therefore, due to the combustion heat which is proportional to the concentrations of CO and H₂ the temperature of the catalytic tank 1 rises to such an extent that HC can be burnt. As a result, the measurement is again interferentially influenced by HC.

Furthermore, it has been proposed to dilute the sample gas SG by the assistant fuel gas J. In such a case, the concentrations of H₂ and CO in the sample gas SG are reduced. Therefore, the combustion heat produced is reduced and the temperature rise of the catalytic tank 1 is likewise reduced. Thus the combustion of HC is hardly promoted. However, disadvantages occur in that the S/N ratio in the water meter 6 is deteriorated due to the above described dilution and thus the accuracy of the measurement is reduced.

From Walter J. Moore "Physical Chemistry", fifth edition, 1972, Longman Group Ltd., London, GB; pages 53 to 55 and 362 to 364 it is already known that burning of hydrogen is an exothermic reaction and therefore, the combustion produces heat. The produced heat of the above reaction, i. e. hydrogen reacts with oxygen, is dependent on the amount of the reactants, e. g. of hydrogen. Therefore, the above dilution of the sample gas containing hydrogen with a dilution gas has been proposed in order to reduce the heat being produced.

It is an object of the present invention to provide a hydrogen gas analyzer capable of conducting a highly accurate measurement without being interferentially influenced by HC.

A hydrogen gas analyzer according to the invention is characterized in that a sample gas supply passage for supplying the sample gas to the catalytic tank and an assistant fuel gas supply passage for supplying the assistant fuel gas to the catalytic tank are branched into a group of parallel passages, respectively; in at least one of the branched passages of each group an on-off valve is provided: and the on-off operation of the on-off valves is interlocked with the switch-over of the measurement ranges of a water meter of the analyzer such that when the concentration of the hydrogen gas in the sample gas introduced into said catalytic tank is comparatively high, a dilution ratio of the sample gas with the assistant fuel gas is increased while in a case when said concentration is comparatively low, said dilution ratio is reduced.

With the above described construction, when the concentration of H₂ in the sample gas is comparatively high, the dilution ratio of the sample gas with the assistant fuel gas is increased, so that the concentrations of H₂ and CO in the sample gas are reduced and thus the combustion heats generated by their combustion are reduced to reduce also the temperature-rise of the catalytic tank, whereby the combustion of HC is hardly promoted. As a result, the interferential influence by HC can be prevented. And, since also the S/N ratio in the water meter is not reduced, the sensitivity of measurement is not reduced and thus a highly accurate measurement can be conducted.

In addition, when the concentration of H₂ in the sample gas is comparatively low, said dilution ratio is reduced but even though this dilution ratio is small, the concentrations of H₂ and CO in the sample gas are small from the beginning and also the combustion heats generated by the combustion are small, so that the combustion of HC is hardly promoted. Accordingly, not only the interferential influence by HC can be prevented but also the S/N ratio in the water meter is not reduced, so that a highly accurate measurement can be conducted.

### BRIEF DESCRIPTION OF THE DRAWINGS

Fig. 1 is a block diagram showing a hydrogen gas analyzer according to one preferred embodiment of the present invention; and
Fig. 2 is a block diagram showing the conventional hydrogen gas analyzer.

### DESCRIPTION OF THE PREFERRED EMBODIMENT

The preferred embodiment of the present invention will be below described with reference to Fig. 1. In addition, the same elements as those in Fig. 2 are designated by the same reference signs.

Referring now to Fig. 1, reference numerals 4A, 4B designate branched passages formed by branching a sample gas supply passage 4 at a point 8. One branched passage 4A being connected with an on-off valve 9, such as an electro-magnetic valve, and a capillary 10 for use in the regulation of flow rate which is in series to the on-off valve 9. The other branched passage 4B being provided with a capillary 11 for use in the regulation of flow rate. Said branched passages 4A, 4B being joined at a point 12 on the downstream side of said capillaries 10, 11.

In addition, reference numerals 5A, 5B designate branched passages formed by branching an assistant fuel gas supply passage 5 at a point 13. One branched passage 5A being connected with an on-off valve 14, such as an electro-magnetic valve, and a capillary 15 for use in the regulation of flow rate which is in series to the on-off valve 14. The other branched passage 5B being provided with a capillary 16 for use in the regulation of flow rate. Said branched passages 5A, 5B being joined at a point 17 on the downstream side of said capillaries 15, 16.

The capillaries 10, 11, 15, 16 have the same flow rate characteristics, in short the quantities of gases passing through the respective capillaries 10, 11, 15, 16 are equal (for example 1 liter/min) to each other. In addition, a gas pressure within the sample gas supply passage 4 on the upstream side of branching point 8 and that within the assistant fuel gas supply passage 5 on the upstream side of branching point 13 are adapted to be constant, respectively.

In order to measure the concentration of H₂ in a sample gas SG by means of the hydrogen gas analyzer having the above described construction, at first, when the concentration of H₂ in the sample gas SG is comparatively high, the on-off valve 9 on the side of the sample gas supply passage 4 is closed and at the same time the on-off valve 14 on the side of the assistant fuel gas supply passage 5 is opened. Whereupon, a ratio of a flow rate Q_{SG} of the sample gas SG at the confluential point 12 in the sample gas supply passage 4 to the flow rate Q_{J} of the assistant fuel gas J at the confluential point 17 in the assistant fuel gas supply passage 5 amounts to 1:2 and thus the sample gas SG supplied to the catalytic tank 1 is diluted with the assistant fuel gas J to reduce the concentration thereof until 1/3 times. Accordingly, the concentrations of H₂ and CO in the sample gas SG are reduced and thus even though they are burnt, the combustion heat is reduced and also the temperature-rise of the catalytic tank 1 is reduced so that the combustion of HC is not promoted. As a result, the interferential influence by HC in the measurement of the concentration of H2 can be prevented. And, since also the S/N ratio in the water meter is not reduced, the sensitivity of measurement is not reduced and a highly accurate measurement can be conducted.

In addition, when the concentration of H₂ in the sample gas SG is comparatively low, said on-off valve 9 is opened and at the same time the on-off valve 14 is closed. Whereupon, said ratio of the flow rate Q_{SG} to the flow rate Q_{J} amounts to 2:1 and thus the sample gas SG supplied to the catalytic tank 1 is diluted with the assistant fuel gas J to reduce the concentration thereof until 2/3 times. In this case, the dilution ratio of the sample gas SG with the assistant fuel gas J is smaller than that in the above described case where the concentration of H₂ is comparatively high but the concentrations of H₂ and CO in the sample gas are small from the beginning and thus the combustion of HC is not promoted, so that not only the interferential influence by HC can be prevented but also the S/N ratio in the water meter 6 is not reduced, whereby the sensitivity of measurement is not reduced and a highly accurate measurement can be conducted.

According to the above described preferred embodiment, the capillaries 10, 11, 15, 16 are adapted to be equal to each other in flow rate characteristic, so that the total gas quantity of the sample gas SG and the assistant fuel gas J introduced into the catalytic tank made constant regardless of the concentration of H₂ in the sample gas SG. Thus no fluctuation occurs in the quantity of the gases supplied to the water meter 6, so that a quantitative determination of H₂O in the water meter 6 can be accurately conducted.

Furthermore, it is preferable that the above described on-off operation of the on-off valves 9, 14 is interlocked with the switch-over of the measurement ranges in the water meter 6. It is sufficient that for example in the case of the higher concentration range the on-off valve 9 is "closed" and the on-off valve 14 is "opened".

As above described, according to the present invention, when the concentration of the hydrogen gas in the sample gas SG introduced into the catalytic tank 1 is comparatively high, the dilution ratio of the sample gas SG with the assistant fuel gas J is increased while when said concentration of the hydrogen gas is comparatively low, said dilution ratio is reduced, so that a highly accurate measurement can be conducted without being interferentially influenced by HC.

## Claims

1. A hydrogen gas analyzer, in which a sample gas (SG) and an assistant fuel gas (J) is introduced into a catalytic tank (1), respectively, to burn said sample gas (SG) and in which a moisture in the resulting gas is quantitatively determined to measure a concentration of a hydrogen gas contained in said sample gas (SG), **characterized in that** a sample gas supply passage (4) for supplying the sample gas (SG) to the catalytic tank (1) and an assistant fuel gas supply passage (5) for supplying the assistant fuel gas (J) to the catalytic tank (1) are branched into a group of parallel passages (4A, 4B; 5A, 5B), respectively; in at least one of the branched passages (4A; 5A) of each group an on-off valve (9; 14) is provided; and the on-off operation of the on-off valves (9; 14) is interlocked with the switch-over of the measurement ranges of a water meter (6) of the analyzer such that when the concentration of the hydrogen gas in the sample gas (SG) introduced into said catalytic tank (1) is comparatively high, a dilution ratio of the sample gas (SG) with said assistant fuel gas (J) is increased while in a case when said concentration is comparatively low, said dilution ratio is reduced.

2. The hydrogen gas analyzer as claimed in claim 1, **characterized in that** each group contains two passages.

3. The hydrogen gas analyzer as claimed in claims 1 or 2, **characterized in that** in each branched passage a capillary (10, 11,15, 16) is provided, said capillaries are adapted to be equal to each other in flow rate characteristic.

## Patentansprüche

1. Wasserstoffgasanalysator, in dem ein Probengas (SG) und ein Hilfsbrenngas (J) in einen katalytischen Behälter (1) eingeleitet werden, um das Probengas (SG) zu verbrennen, und in dem der Dampf im sich ergebenden Gas quantitativ bestimmt wird, um die Konzentration des im Probengas (SG) enthaltenen Wasserstoffgases zu messen, **dadurch gekennzeichnet,** daß ein Probengas-Zuführkanal (4) zum Zuführen des Probengases (SG) zum katalytischen Behälter (1) und ein Hilfsbrenngas-Zuführkanal (5) zum Zuführen des Hilfsbrenngases (J) zum katalytischen Behälter (1) in eine Gruppe paralleler Kanäle (4A, 4B; 5A, 5B) verzweigt sind; in mindestens einem der Zweigkanäle (4A; 5A) jeder Gruppe ein Ein/Aus-Ventil (9; 14) vorhanden ist und der Ein/Aus-Betrieb der Ein/Aus-Ventile (9; 14) mit dem Umschalten der Meßbereiche eines Wassermeßgeräts (6) des Analysators so gekoppelt ist, daß dann, wenn die Konzentration von Wasserstoffgas in dem in den katalytischen Behälter (1) eingeleiteten Probengas (SG) vergleichsweise hoch ist, das Verdünnungsverhältnis des Probengases (SG) mit dem Hilfsbrenngas (J) erhöht ist, während dann, wenn die Konzentration vergleichsweise niedrig ist, das Verdünnungsverhältnis verringert wird.

2. Wasserstoffgasanalysator nach Anspruch 1, **dadurch gekennzeichnet,** daß jede Gruppe zwei Kanäle enthält.

3. Wasserstoffgasanalysator nach einem der Ansprüche 1 oder 2, **dadurch gekennzeichnet,** daß in jedem Zweigkanal eine Kapillare (10, 11, 15, 16) vorhanden ist, die so ausgebildet sind, daß sie jeweils gleiche Eigenschaften hinsichtlich der Strömungsrate aufweisen.

## Revendications

1. Un appareil pour analyser l'hydrogène gazeux, dans lequel respectivement, un gaz à analyser (SG) et un gaz combustible d'assistance (J) sont introduits dans un réservoir catalytique (1), pour brûler ledit gaz à analyser (SG) et dans lequel l'humidité présente dans le gaz résultant est déterminée quantitativement pour mesurer la concentration en hydrogène gazeux contenu dans ledit gaz à analyser (SG), caractérisé en ce qu'un passage (4) d'alimentation en gaz à analyser pour amener le gaz à analyser (SG) au réservoir catalytique (1) et un passage (5) d'alimentation en gaz combustible d'assistance pour amener le gaz combustible d'assistance (J) au réservoir catalytique (1) sont branchés respectivement en un groupe de passages parallèles (4A, 4B; 5A, 5B); en ce qu'une valve d'ouverture-fermeture (9; 14) est prévue sur au moins l'un des passages de branchement (4A; 5A) de chacun des groupes; et en ce que le fonctionnement d'ouverture-fermeture des valves d'ouverture-fermeture (9; 14) est synchronisé avec la commutation des gammes de mesure d'un organe de mesure d'eau (6) de l'appareil pour analyser, de telle façon que lorsque la concentration en hydrogène gazeux du gaz à analyser (SG) introduit dans ledit réservoir catalytique (1) est comparativement élevée, le rapport de dilution du gaz à analyser (SG) dans ledit gaz combustible d'assistance (J) soit augmenté, tandis que dans le cas où ladite concentration est comparativement faible, ledit rapport de dilution soit réduit.

2. L'appareil pour analyser l'hydrogène gazeux tel que revendiqué à la revendication 1, caractérisé en ce que chaque groupe contient deux passages.

3. L'appareil pour analyser l'hydrogène gazeux tel que revendiqué dans les revendications 1 ou 2, caractérisé en ce que, dans chaque passage de branchement, est prévu un tube capillaire (10, 11, 15, 16), chacun desdits tubes capillaires étant susceptible d'être égal à chacun des autres, en ce que qui concerne les caractéristiques de débit d'écoulement.
